# FASCICULE DE BREVET EUROPEEN

(11) **EP 0 762 106 B1**
(45) Date de publication et mention de la délivrance du brevet: **23.10.2002**
(21) Numéro de dépôt: 96401694.3
(22) Date de dépôt: 30.07.1996
(51) Int. Cl.: G01N 7/14

(54) **Procédé de détermination du taux de gaz dissous dans un milieu diphasique**
Verfahren zur Bestimmung des gelösten Gasverhältnisses in einem zweiphasigen Medium
Method fo determining ratio of dissolved gas in a diphasic medium

(30) Priorité: 11.09.1995 FR 9510696
(43) Date de publication de la demande: 12.03.1997
(73) Titulaire: INSTITUT FRANCAIS DU PETROLE, 92500 Rueil Malmaison (FR)
(72) Inventeur: Porot, Pierre, 75010 Paris (FR)

(56) Documents cités:
- DE-A- 4 400 385
- DE-B- 2 362 158
- DE-U- 7 344 322
- FR-A- 2 670 894
- US-A- 4 289 018
- US-A- 5 442 948

## Description

La présente invention concerne le domaine des mesures plus particulièrement destinées à déterminer un taux de gaz dans un milieu diphasique gaz-liquide.

Plus précisément la présente invention vise à connaître le taux de gaz dissous dans un milieu diphasique gaz-liquide.

Il est connu de déterminer un tel taux grâce à un appareil spécifique tel qu'une pompe à vide qui crée une détente du gaz puis l'on mesure le volume ou la variation de volume par tout moyen connu en soi afin d'atteindre le taux de gaz dissous initialement dans le mélange. Bien entendu la pompe à vide a ici pour seule fonction de diminuer la pression du mélange, et elle peut effectivement créer un vide poussé, de plusieurs torrs par exemple. Avec un tel moyen, la fonction détente du mélange est correctement réalisée mais le coût du circuit sur lequel il est monté est plus élevé.

Il est également connu de déterminer le taux global de gaz contenu dans un échantillon lors d'une opération de désorption du gaz, soit par un phénomène de cavitation de cet échantillon et de mesure du gaz ainsi désorbé, tel que cela est décrit dans le document DE-U-7344322, soit par détente de cet échantillon puis par compression de ce même échantillon, ce qui permet de déterminer ainsi le taux global de gaz, comme cela est mieux explicité dans le document US-A-5442948.

Ces types de détermination du taux de gaz ne permettent que de relever le taux global du gaz présent dans l'échantillon, c'est à dire le gaz présent à l'état gazeux, généralement sous forme de bulles, et le gaz dissous dans cet échantillon.

Le taux de gaz dissous dans un mélange est un paramètre très important par exemple dans des circuits huile de moteurs car dès qu'il existe des pertes de charge une détente se produit dans le circuit, l'air dissous désorbe, devient gazeux. Autrement dit, l'air dissous dans le circuit d'huile moteur peut s'avérer tout à fait néfaste pour le fonctionnement de ce circuit. L'huile moteur qui se dégrade perd ses capacités lubrifiantes.

Par ailleurs l'ajout sur les moteurs courants d'un moyen spécifique (tel une pompe à vide) pour obtenir le taux d'air dissous, est exclu par les motoristes du fait du surcoût entraîné par cet ajout.

En outre, la commande d'un tel moyen rend le système plus compliqué.

Enfin l'encombrement lié à l'ajout d'un élément n'est pas favorable notamment s'il s'agit d'une application dans un moteur.

Il existe donc un besoin pour déterminer de façon simple, fiable et peu (voire pas) coûteux le taux de gaz dissous dans un mélange diphasique.

Plus précisément, il s'agit de déterminer le risque maximum c'est-à-dire le taux maximum de gaz dissous capable, sous certaines conditions, de devenir gazeux. Il devient dès lors judicieux de placer un échantillon dans les pires conditions réellement rencontrées afin d'en obtenir le taux maximum de gaz dissous.

La présente invention propose une solution à ces problèmes jusqu'ici non résolus de façon satisfaisante.

Ainsi la présente invention a pour objet un procédé de détermination du taux de gaz dissous dans un échantillon diphasique, comprenant les actions suivantes :
- faire désorber le gaz initialement dissous dans l'échantillon et mesurer le taux global de gaz contenu dans cet échantillon
- mesurer le taux de gaz présent à l'état gazeux dans l'échantillon avant désorption
- comparer la mesure du taux global de gaz avec la mesure du taux de gaz présent à l'état gazeux et en déduire le taux de gaz dissous dans ledit mélange diphasique.

Selon un mode de réalisation de l'invention, l'étape de désorption comprend une détente du gaz.

Conformément à un autre mode de réalisation de l'invention, l'étape de désorption comprend une variation de la température du gaz.

De façon particulière, la détente et la mesure du taux global de gaz sont exécutés dans un même dispositif en, successivement, diminuant puis augmentant la pression de l'échantillon.

Avantageusement, la mesure du taux de gaz présent à l'état gazeux est réalisée en utilisant le même dispositif que celui réalisant la détente et la mesure du taux global de gaz.

Préférentiellement la désorption pour l'échantillon est réalisée dans les conditions les plus défavorables susceptibles d'être rencontrées par le mélange dans un circuit réel.

Plus particulièrement, la désorption de l'échantillon est réalisée à la pression minimale susceptible d'exister dans le circuit réel.

Une application intéressante de l'invention concerne la détermination du taux d'air dissous dans le circuit d'huile d'un moteur.

De ce qui précède, il ressort que l'on utilise un même moyen ou plus précisément la réversibilité du moyen, pour obtenir une information supplémentaire à savoir le taux de gaz dissous.

Par ailleurs la présente invention permet d'obtenir des mesures très précises.

Une façon de mettre en oeuvre l'invention peut consister à prendre un échantillon du milieu diphasique, de toute manière connue en soi. Éventuellement faire une mesure du taux de gaz présent initialement dans le mélange échantillonné. Puis il s'agit de faire désorber le gaz initialement dissous par exemple par une détente ou encore par une variation de température qui elle-même crée une détente du gaz.

Ayant déterminé la valeur globale du taux de gaz dans le mélange diphasique, il faut comparer cette valeur à la valeur du taux de gaz présent à l'état gazeux c'est-à-dire avant désorption.

On atteint ainsi précisément la valeur du taux de gaz dissous dans le mélange.

Toute méthode et/ou tout moyen connu en soi peut être utilisé pour mesurer les taux de gaz évoqués ci-avant.

Cependant, de façon particulière, la détente et la compression de l'échantillon peuvent être réalisés successivement par un même dispositif.

Il semble nécessaire d'attendre un temps "de repos" entre la détente et la compression nécessaires à la mesure, afin que la désorption soit terminée lorsque la mesure est effectuée.

Selon un exemple préféré, ce dispositif peut consister en une chambre d'échantillon associée à un piston. Le piston, pouvant assurer l'augmentation ou la diminution du volume de la chambre, crée ainsi successivement une détente par l'augmentation de volume de la chambre puis une compression par diminution du volume.

Préférentiellement la compression de l'échantillon est réalisée de manière régulière.

Il était connu jusqu'à présent, notamment par le brevet français FR 2 670 894, de déterminer le taux de gaz présent dans un liquide grâce à une compression d'un échantillon, en relevant le point d'inflexion de la courbe de la variation de volume en fonction de la variation de pression.

La présente invention permet avantageusement d'utiliser une telle détermination du taux de gaz présent à l'état gazeux par le dispositif précité et d'en obtenir l'information supplémentaire constituée par le taux de gaz dissous dans le milieu diphasique échantillonné.

En effet, connaissant par cette première mesure le taux de gaz présent dans le milieu, il suffit selon l'invention de faire désorber l'échantillon (par exemple en créant une détente), puis de mesurer le taux de gaz de l'échantillon ainsi désorbé.

En comparant ce dernier taux de gaz avec le taux de gaz connu de la première mesure, on peut en déduire le taux de gaz dissous dans le milieu.

Avec le dispositif susnommé connu et en utilisant le procédé selon l'invention, il est possible de faire descendre la pression jusqu'à 0,4 bar ou 0,5 bar. Ces valeurs correspondent aux pressions minimales susceptibles d'être créées dans des circuits d'huile moteur. Ces chutes de pression existent notamment dans les paliers. Ces valeurs déterminées selon l'invention sont donc tout à fait représentatives du taux de gaz capable d'être désorbé dans un tel circuit. Cette information est de ce fait très appréciée des motoristes.

De ce qui précède, il ressort que la présente invention apporte une solution simple, fiable à un problème réel. L'information supplémentaire donnée par l'invention correspond à une attente des utilisateurs.

Sans sortir du cadre de l'invention, d'autres applications de l'invention peuvent être citées notamment dans les circuits du liquide de refroidissement des moteurs, ou dans d'autres échangeurs de chaleur.

Plus généralement, l'invention s'applique à tout circuit dans lequel on redoute une chute de pression du mélange en circulation.

## Revendications

1. Procédé de détermination du taux de gaz dissous dans un échantillon diphasique, **caractérisé en ce qu'**il comprend les actions consistant à :
- faire désorber le gaz initialement dissous dans l'échantillon et mesurer le taux global de gaz contenu dans cet échantillon
- mesurer le taux de gaz présent à l'état gazeux dans l'échantillon avant désorption
- comparer la mesure du taux global de gaz avec la mesure du taux de gaz présent à l'état gazeux et en déduire le taux de gaz dissous dans ledit échantillon diphasique.

2. Procédé selon la revendication 1, **caractérisé en ce que** l'étape de désorption comprend une détente du gaz.

3. Procédé selon la revendication 1, **caractérisé en ce que** l'étape de désorption comprend une variation de température du gaz.

4. Procédé selon la revendication 2, **caractérisé en ce que** la détente et la mesure du taux global de gaz sont exécutés dans un même dispositif en, successivement, diminuant puis augmentant la pression de l'échantillon.

5. Procédé selon la revendication 1 en combinaison avec la revendication 4, **caractérisé en ce que** la mesure du taux de gaz présent à l'état gazeux est réalisée en utilisant le même dispositif que celui réalisant la détente et la mesure du taux global de gaz.

6. Procédé selon l'une quelconque des revendications précédentes, **caractérisé en ce que** la désorption de l'échantillon est réalisée à la pression minimale susceptible d'exister dans le circuit réel.

7. Application du procédé selon l'une quelconque des revendications précédentes à la détermination du taux d'air dissous dans le circuit d'huile d'un moteur.

## Claims

1. Method for determining the ratio of gas dissolved in a diphasic sample, **characterised in that** it comprises the actions consisting in:
- desorbing the gas initially dissolved in the sample and measuring the overall ratio of gas contained in this sample,
- measuring the ratio of gas present in the gaseous state in the sample before desorption,
- comparing the measurement of the overall ratio of gas with the measurement of the ratio of gas present in the gaseous state and deducing therefrom the ratio of gas dissolved in said diphasic sample.

2. Method according to claim 1, **characterised in that** the desorption step comprises expansion of the gas.

3. Method according to claim 1, **characterised in that** the desorption step comprises variation of the temperature of the gas.

4. Method according to claim 2, **characterised in that** the expansion and the measurement of the overall ratio of gas are carried out in a same device by successively reducing then increasing the pressure of the sample.

5. Method according to claim 1 in combination with claim 4, **characterised in that** the measurement of the ratio of gas present in the gaseous state is carried out using the same device as that carrying out the expansion and the measurement of the overall ratio of gas.

6. Method according to any one of the preceding claims, **characterised in that** the desorption of the sample is carried out at the minimum pressure likely to exist in the real circuit.

7. Application of the method according to any one of the preceding claims to determining the ratio of air dissolved in the oil circuit of an engine.

## Patentansprüche

1. Verfahren zur Bestimmung des Mengenverhältnisses von Gasen, die in einer biphasischen Probe gelöst sind, **dadurch gekennzeichnet, dass** es die Schritte umfasst, die darin bestehen,
- das anfangs in der Probe gelöste Gas desorbieren zu lassen und das Gesamtmengenverhältnis an in dieser Probe enthaltenem Gas zu messen
- das Mengenverhältnis von vor der Desorption in gasförmigen Zustand in der Probe vorliegendem Gas zu messen
- die Messung des Gasgesamtmengenverhältnisses mit der Messung des Mengenverhältnisses von in gasförmigen Zustand in der Probe vorliegendem Gas zu vergleichen und davon das Mengenverhältnis von in der biphasischen Probe gelöstem Gas abzuleiten.

2. Verfahren nach Anspruch 1, **dadurch gekennzeichnet, dass** der Desorptionsschritt eine Stufe der Gasentspannung umfasst.

3. Verfahren nach Anspruch 1, **dadurch gekennzeichnet, dass** der Desorptionsschritt eine Stufe der Variation der Temperatur des Gases umfasst.

4. Verfahren nach Anspruch 2, **dadurch gekennzeichnet, dass** die Entspannung und die Messung des Gasgesamtmengenverhältnisses in derselben Vorrichtung unter fortschreitender Verminderung und dann Erhöhung des Probendrucks durchgeführt werden.

5. Verfahren nach Anspruch 1 in Kombination mit Anspruch 4, **dadurch gekennzeichnet, dass** die Messung des Mengenverhältnisses von in gasförmigen Zustand vorliegendem Gas unter Verwendung derselben Vorrichtung wie jener durchgeführt wird, die die Entspannung und die Messung des Gasgesamtmengenverhältnisses durchführt.

6. Verfahren nach einem der vorhergehenden Ansprüche, **dadurch gekennzeichnet, dass** die Desorption der Probe bei dem geringsten Druck durchgeführt wird, der in dem realen Kreislauf existieren kann.

7. Verwendung des Verfahrens nach einem der vorhergehenden Ansprüche, bei der Bestimmung des Mengenverhältnisses von Luft, die in einem Ölkreislauf eines Motors gelöst ist.
